Europäisches Patentamt

European Patent Office

Office européen des brevets

(19)

(11) Publication number: **0 275 930 B1**

# EUROPEAN PATENT SPECIFICATION

(12)

(45) Date of publication of patent specification: **02.01.92**

(51) Int. Cl.5: **C10G 29/00**, B01J 8/24, C07C 2/00

(21) Application number: **88100474.1**

(22) Date of filing: **14.01.88**

(54) **Upgrading diene-containing hydrocarbons.**

(30) Priority: **23.01.87 US 6408**
**23.01.87 US 6399**
**23.01.87 US 6089**

(43) Date of publication of application:
**27.07.88 Bulletin 88/30**

(45) Publication of the grant of the patent:
**02.01.92 Bulletin 92/01**

(84) Designated Contracting States:
**BE DE FR GB IT NL**

(56) References cited:
EP-A- 0 093 477       EP-A- 0 262 049
US-A- 4 097 367       US-A- 4 417 086
US-A- 4 417 087       US-A- 4 547 616

(73) Proprietor: **MOBIL OIL CORPORATION**
**3225 Gallows Road**
**Fairfax, Virginia 22037-0001(US)**

(72) Inventor: **Avidan, Amos Andrew**
**2120 Stackhouse Drive**
**Yardley Pennsylvania 19067(US)**
Inventor: **Smith, Fritz Arthur**
**109 Cedar Glen Drive**
**New Hope Pennsylvania 19838(US)**
Inventor: **Beech, James Harding, Jr.**
**2205 Charwood Drive**
**Wilmington Delaware 19810(US)**
Inventor: **Tabak, Samuel Allen**
**204 East Pine Street**
**Wenonah New Jersey 08090(US)**

(74) Representative: **Kador & Partner**
**Corneliusstrasse 15**
**W-8000 München 5(DE)**

EP 0 275 930 B1

Rank Xerox (UK) Business Services

**Description**

This invention relates to a process for upgrading olefin streams rich in dienes to heavier hydrocarbons rich in aliphatics and aromatics.

Developments in zeolite catalysis and hydrocarbon conversion processes have created interest in utilizing olefinic feedstocks for producing $C_5^+$ gasoline, diesel fuel, etc. In addition to basic chemical reactions promoted by ZSM-5 type zeolite catalysts, a number of discoveries have contributed to the development of new industrial processes. These are safe, environmentally acceptable processes for utilizing feedstocks that contain olefins. Conversion of $C_2$-$C_4$ alkenes and alkanes to produce aromatics-rich liquid hydrocarbon products were found by Cattanach (US 3,760,024) and Yan et al (US 3,845,150) to be effective processes using the ZSM -5 type zeolite catalysts. In U.S. Patents 3,960,978 and 4,021,502, Plank, Rosinski and Givens disclose conversion of $C_2$-$C_5$ olefins, alone or in admixture with paraffinic components, into higher hydrocarbons over crystalline zeolites having controlled acidity. Garwood et al. have also contributed to the understanding of catalytic olefin upgrading techniques and improved processes as in U.S. Patents 4,150,062, 4,211,640 and 4,227,992.

Conversion of olefins, especially propene and butenes, over HZSM-5 is effective at moderately elevated temperatures and pressures. The conversion products are sought as liquid fuels, especially the $C_5^+$ aliphatic and aromatic hydrocarbons and $C_4$ hydrocarbons, in particular iso-butane. Product distribution for liquid hydrocarbons can be varied by controlling process conditions, such as temperature, pressure and space velocity. Gasoline ($C_5$-$C_{10}$) is readily formed at elevated temperature e.g., up to about 700° C. and moderate pressure from ambient to about 5500 kPa, preferably about 200 to 2900 kPa. Olefinic gasoline can be produced in good yield and may be recovered as a product or fed to a low severity, high pressure reactor system for further conversion to heavier distillate-range products. Distillate mode operation can be employed to maximize production of $C_{10}^+$ aliphatics by reacting the lower and intermediate olefins at high pressure and moderate temperature. Operating details for such oligomerization units are disclosed in U.S. Patents 4,456,779; 4,497,968 (Owen et al.) and 4,433,185 (Tabak). At moderate temperature and relatively high pressure, the conversion conditions favor distillate-range product having a normal boiling point of at least 165° C. (330° F.).

Many feedstocks of commercial interest, such as thermal cracking byproduct, etc., contain both mono-olefins and diolefins (e.g. $C_2$-$C_6$ mono-alkenes and $C_4^+$ dienes) along with $C_1$-$C_{10}$ light aliphatics, and a minor amount of aromatics. Gaseous and liquid streams containing dienes are typically produced in thermal cracking operations. One common example is pyrolysis gasoline, which is produced as ethene (ethylene) byproduct. Such diene-containing streams are often difficult to process due to poor thermal stability and the tendency of dienes to form coke and gum deposits. This complicates preheating of such streams into the high temperatures required of most catalytic upgrading processes. Prior attempts to upgrade such materials have pretreated the feedstock to hydrogenate the diolefin selectively, as in U.S. Patent No. 4,052,477 (Ireland et al). The present invention is concerned with providing a safe and low cost technique for catalytically converting diene-rich streams to high value $C_4^+$ products

It has been found that diene-containing olefinic light hydrocarbons can be upgraded directly to liquid hydrocarbons rich in $C_5^+$ aliphatics and aromatics by catalytic conversion. This technique is particularly useful for upgrading $C_4^+$ liquid pyrolysis products, which may contain minor amounts of ethene, propene, $C_2$-$C_4$ paraffins and hydrogen produced in cracking petroleum fractions, such as naphtha, ethane or the like. By upgrading the complex olefinic by-product, gasoline yield and/or aromatics production of cracking units can be significantly increased.

Accordingly, the present invention provides a process for converting an olefinic feed containing 5 to 90 wt% $C_4$-$C_6$ olefins and at least 1 wt% dienes to a liquid product, of reduced diene content, comprising $c_4^+$ hydrocarbons and having a $C_3$-$C_5$ alkane:alkene weight ratio of 0.2 to 200, said process comprising contacting said feed with a gas-fluidized bed of a catalyst comprising a zeolite having a constraint index of 1 to 12 at a temperature of 220 to 510° C and a reaction severity index below 50, the feed being at a temperature below that of the bed before introduction thereinto.

FIG. 1 is a schematic view of a fluidized bed reactor system of the present invention;
FIG. 2 is a vertical cross section view of a liquid-gas feed nozzle which is employed to introduce low temperature diene feed into the reactor bed;
FIG. 3 is an aging plot showing the effect of adding butadiene to a $C_2$-$C_4$ olefinic feed.
FIG. 4 is a schematic view of a fluidized bed reactor and regeneration system of the invention;
FIG. 5 is a process flow sheet for converting olefin feedstock to aromatics-rich product, showing a reactor and effluent separation equipment.

Catalysts

Recent developments in zeolite technology have provided a group of medium pore siliceous materials having similar pore geometry. Most prominent among these intermediate pore size zeolites is ZSM-5, which is usually synthesized with Bronsted acid active sites by incorporating a tetrahedrally coordinated metal, such as Al, Ga, B, Fe or mixtures thereof, within the zeolitic framework. These medium pore zeolites are favored for acid catalysts; however, the advantages of ZSM-5 structures may be utilized by employing highly siliceous materials or crystalline metallosilicate having one or more tetrahedral species having varying degrees of acidity. ZSM-5 crystalline structure is readily recognized by its X-ray diffraction pattern, which is described in U.S. Patent No. 3,702,866 (Argauer, et al.).

The oligomerization catalysts preferred for use herein include the medium pore (i.e., about 7-5A) zeolites having a silica-to-alumina ratio of at least 12, a constraint index of 1 to 12 and acid cracking activity (alpha value) of 10-250, more preferably 10 to 80 based on total catalyst weight. In the fluidized bed reactor the coked catalyst may have an apparent activity (alpha value) of 10 to 80 under the process conditions to achieve the required degree of reaction severity.

Representative zeolites are ZSM-5, ZSM-11, ZSM-12, ZSM-22, ZSM-23, ZSM-35, ZSM-38, and ZSM-48. Other suitable zeolites are disclosed in U.S. Patents 3,709,979; 3,832,449; 4,076,979; 3,832,449; 4,076,842; 4,016,245 and 4,046,839; 4,414,423; 4,417,086; 4,517,396 and 4,542,251. While suitable zeolites having a coordinated metal oxide to silica molar ratio of 20:1 to 200:1 or higher may be used, it is advantageous to use ZSM-5 having a silica:alumina molar ratio of about 25:1 to 70:1, suitable modified if desired to adjust acidity and aromatization characteristics.

These zeolites may be employed in their acid forms, ion exchanged or impregnated with one or more suitable metals, such as Ga, Pd, Zn, Ni, Co and/or other metals of Periodic Groups III to VIII. The zeolite may include a hydrogenation-dehydration component (sometimes referred to as a hydrogenation component) which is generally one or more metals of group IB, IIB, IIIB, VA, VIA or VIIIA of the Periodic Table (IUPAC), especially aromatization metals, such as Ga, Pd, etc. Useful hydrogenation components include the noble metals of Group VIIIA, especially platinum, but other noble metals, such as palladium, gold, silver, rhenium or rhodium, may also be used. Base metal hydrogenation components may also be used, especially nickel, cobalt, molybdenum, tungsten, copper or zinc. The catalyst materials may include two or more catalytic components, such as a metallic oligomerization component (e.g., ionic $Ni^{+2}$, ans a shape-selective medium pore acidic oligomerization catalyst, such as ZSM-5 zeolite) which components may be present in admixture or combined in a unitary bifunctional solid particle. It is possible to utilize an ethene dimerization metal or oligomerization agent to effectively convert feedstock ethene in a continuous reaction zone.

Certain of the ZSM-5 type medium pore shape selective catalysts are sometimes known as pentasils. In addition to the preferred aluminosilicates, the borosilicate, ferrosilicate and "silicalite" materials may be employed.

ZSM-5 type pentasil zeolites are particularly useful in the process because of their regenerability, long life and stability under the extreme conditions of operation. Usually the zeolite crystals have a crystal size from about 0.01 to 2 microns or more. In order to obtain the desired particle size for fluidization in the turbulent regime, the zeolite catalyst crystals are preferably bound with a suitable inorganic oxide, such as silica, alumina, etc. to provide a zeolite concentration of about 5 to 95 wt.%. It is advantageous to employ ZSM-5 having a silica:alumina molar ratio of 25:1 or greater in a once-through fluidized bed unit to convert 60 to 100 percent, preferably at least 75 wt %, of the monoalkenes and dienes in the feedstock. In the description, a 25% H-ZSM-5 catalyst calcined with 75% silica-alumina matrix binder is employed unless otherwise stated.

FLUIDIZED BED REACTION ZONE

It is essential that the diolefin containing feed contact a fluidized bed of catalyst. Fixed catalyst beds will not work in the process of the present invention.

Fluidization is a complex phenomenon. In general terms, suitable fluidized beds include both dense phase beds and dilute phae beds. The lower and upper limits of fluidization will be discussed.

In an expanded bed, there is enough of an increase in up-flowing gas rate to cause particles to move apart and a few even vibrate and move about in restricted regions. An expanded bed is usually not suitable for use herein.

An ebullating bed represents the lower threshold of fluidization required for use in the present invention. An ebullating bed permits just enough circulation to work. Any gum deposited on the catalyst might deactivate the catalyst near the feed inlet momentarily, but there would be enough gross circulation within the reactor so that incoming feed would see active catalyst. The gummed up catalyst could circulate to other portions of the ebullating bed and, in time, the gum components would be cracked and removed, in situ. A portion of the catalyst could also be continuously withdrawn and replaced with fresh or regenerated catalyst.

A conventional dense phase fluidized bed is preferred. Gas-solid systems in this stage are also called aggregative fluidized beds, a heterogeneously fluidized bed, a bubbling fluidized bed, or simply a gas fluidized bed.

An especially preferred gas-fluidized bed is the turbulent fluidized bed disclosed in US 4,547,616. Such a turbulent fluidized bed is characterized by vigorous solid mixing, and strong interations between the gas and solid phases.

Dilute phase fluidized beds represent an upper limit on fluidization and are also suitable for use herein. This type of fluidized bed is also referred to as a disperse-, dilute-, or lean-phase fluidized bed. Such beds occur when the superficial vapor velocity in the bed exceeds the terminal velocity of the solids in the bed.

It is also possible, and may be very beneficial, to combine one or more types of fluidized beds for use in the present invention. Specifically, all or a portion of the feed can be added to a dilute phase riser which discharges into a dense phase bed. In another embodiment, liquid feed can be added to a relatively dense phase bed over which is a dilute phase transport riser which discharges into another dense bed of catalyst or into catalyst/product separation means.

In all embodiments, the feed addition means has a low enough temperature/residence time so that plugging of the feed distributor or feed nozzle is avoided, and feed sees an overwhelming amount of hot, active catalyst. Undoubtedly, some dienes form gum on the catalyst, but because the bed is fluidized no plugging of the bed can occur. The very active zeolite catalyst will eventually crack much of the gum, or gum precursor material, to lighter products in the fluidized bed reactor.

Particle size distribution can be a significant factor in fluidization. It is desired to operate the process with particles that will mix well throughout the bed. Large particles having a particle size greater than 250 microns should be avoided, and it is advantageous to employ a particle size range consisting essentially of 1 to 150 microns. Average particle size is usually 20 to 100 microns, preferably 40 to 80 microns. Particle distribution may be enhanced by having a mixture of larger and smaller particles within the operative range, and it is particularly desirable to have a significant amount of fines. Close control of distribution can be maintained to keep about 10 to 25 wt % of the total catalyst in the reaction zone in the size range less than 32 microns. The size range of fluidizable particles is classified as Geldart Group A. The fluidization regime is preferably controlled to assure operation between the transition velocity and transport velocity, and these fluidization conditions are substantially different from those found in non-turbulent dense beds or transport beds.

In the discussion that follows, percents are by weight unless otherwise stated.

Feedstocks - Reaction Conditions

Suitable olefinic feedstocks comprise $C_4$-$C_6$ alkenes including conjugated dienes such as 1,3-butadiene, pentadiene isomers, hexadienes, cyclic dienes, or similar $C_4^+$ aliphatic liquid hydrocarbons having diethylenic conjugated unsaturation. Aromatics coproduced with the liquid olefinic components may be cofed or separated by solvent extraction prior to conversion of the diene-rich feedstock. Non-deleterious components, such as paraffins and inert gases, may be present. A particularly useful feedstock is a liquid by-product of pyrolysis or thermal cracking units containing typically 40-95 wt % $C_4$-$C_6$ total mono-olefins and di-olefins, including about 5-60 wt. % diene, along with varying amounts of $C_3$-$C_8$ paraffins, aromatics and inerts. Specific examples are given in Table 1 below. The processes tolerates a wide range of lower alkanes, from 0 to 95%. Preferred pyrolysis feedstocks contain more than 50 wt % $C_4$-$C_6$ lower aliphatic hydrocarbons, and contain sufficient olefins to provide an olefinic partial pressure of at least 50 kPa. Under the high severity reaction conditions employed in the present invention, lower alkanes may be partially converted to heavier hydrocarbonds

The reaction severity conditions can be controlled to optimize yield of $C_4$-$C_9$ hydrocarbons or of $C_6$-$C_8$ aromatics. It is understood that aromatics and light paraffin production is promoted by those zeolite catalysts having a high concentration of Bronsted acid reaction sites. Accordingly, an important criterion is selecting and maintaining catalyst inventory to provide either fresh or regenerated catalyst having the desired properties. Typically, acid cracking activity (alpha value) can be maintained from high activity values greater than 100 or 200 to significantly lower values under steady state operation by controlling catalyst deactivation and regeneration rates to provide an apparent average alpha value below 100, preferably 10 to 80.

Reaction temperatures and contact time are also significant factors in the reaction severity. The reaction severity index (R.I.) is the weight ratio of propane to propene in reactor effluent. While this index may vary from about 0.2 to 200, it is preferred below about 50, with optimum operation at 0.7 to 2 in the substantial absence of added propane. While reaction severity is advantageously determined by the weight ratio of propane:propene in the gaseous phase, it may also be approximated by the analogous ratios of butanes:butenes, pentanes:pentenes, or the average of total reactor effluent alkanes:alkenes in the C3-C5 range. Accordingly, these alternative expressions may be a more accurate measure of reaction severity conditions when propane is added to the feedstock. The optimal value will depend upon the exact catalyst composition, feedstock and reaction conditions; however, the typical diene-rich feed mixtures used in the examples herein and additional olefinic feeds can be optimally upgraded to the desired aliphatic-rich gasoline by keeping the R.I. at about 1.

Upgrading olefins with hydrogen contributors in fluidized bed cracking and oligomerization units is taught by Owen et al in U. S. Patent 4,090,949. This technique is particularly useful for operation with a pyrolysis cracking unit to increase overall production of liquid product.

The use of fluidized bed catalysis permits the conversion system to be operated at low pressure drop, which in an economically practical operation can provide a maximum operating pressure only 50 to 200 kPa above atmospheric pressure. Somewhat higher pressures, up to 2500 kPa may be used to favor aromatics production. Close temperature control is possible by turbulent regime operation. The temperature can be maintained within close tolerances, often less than 5°C. Except for a small zone adjacent the bottom gas inlet, the midpoint measurement is representative of the entire bed, due to the thorough mixing achieved.

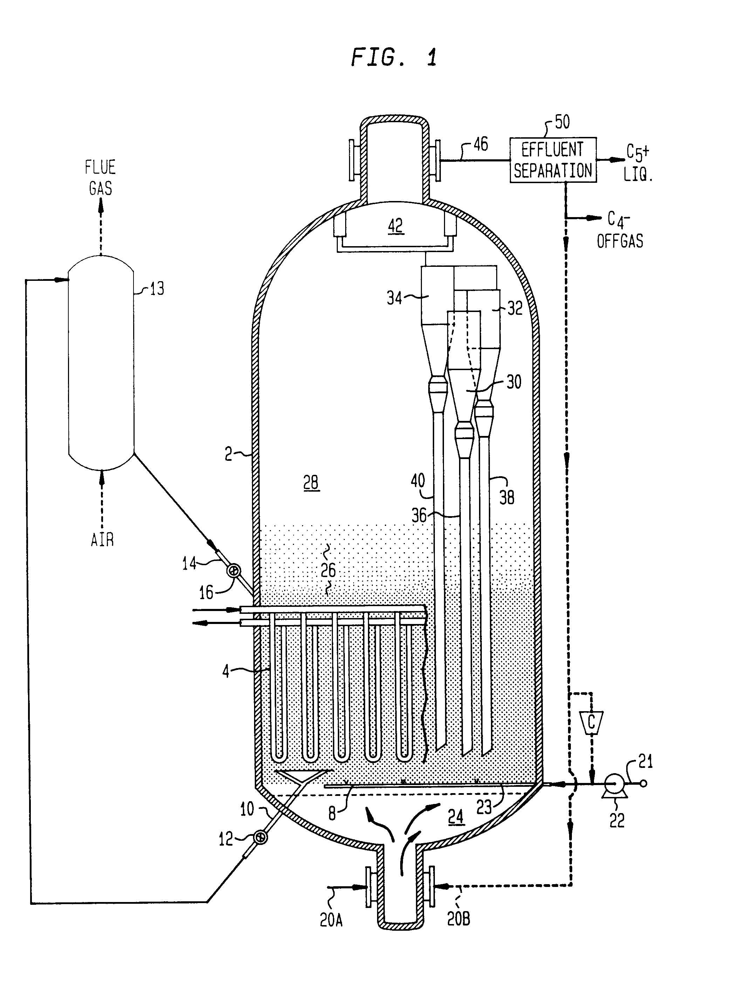

Referring now to FIG. 1, a reactor vessel 2 is shown provided with heat exchange tube means 4. There may be several separate heat exchange steam generating tube bundles so that temperature control can be separately exercised over the fluid catalyst bed. The bottoms of the tubes are spaced above a feed distributor grid 8 sufficiently to be free of jet action by the charged gas passing through the small diameter holes in the grid 8. Although depicted without baffles, the vertical reaction zone can contain open end tubes above the grid for maintaining hydraulic constraints, as disclosed in US Pat. 4,251,484 (Daviduk and Haddad). Optionally, a variety of horizontal baffles may be added to limit axial mixing in the reactor. Heat released from the reaction can be controlled by adjusting feed temperature in a known manner. A large portion of reaction heat can be removed by feeding cold liquid into the reactor at a temperature below average bed temperature preferably at least 200°C below. In the reactor configuration shown in the heat exchanger tubes can function to limit mixing in the reactor.

The system provides for withdrawing catalyst from above grid 8 by conduit means 10. This flow line can be provided with control valve means 12 for passage to catalyst regeneration invessel 13, where cooked catalyst particles are oxidatively regenerated in contact with air or other regeneration gas at high temperature. The oxidatively regenerated catalyst is then passed to the reactor fluid bed of catalyst by conduit means 14 and flow control valve 16. The regenerated catalyst is charged to the catalyst bed sufficiently below the upper interface to achieve good mixing in the fluid bed. Since the flow of regenerated catalyst passed to the reactor can be relatively small, hot regenerated catalyst does not ordinarily upset the temperature constraints of the reactor operations in a significant amount.

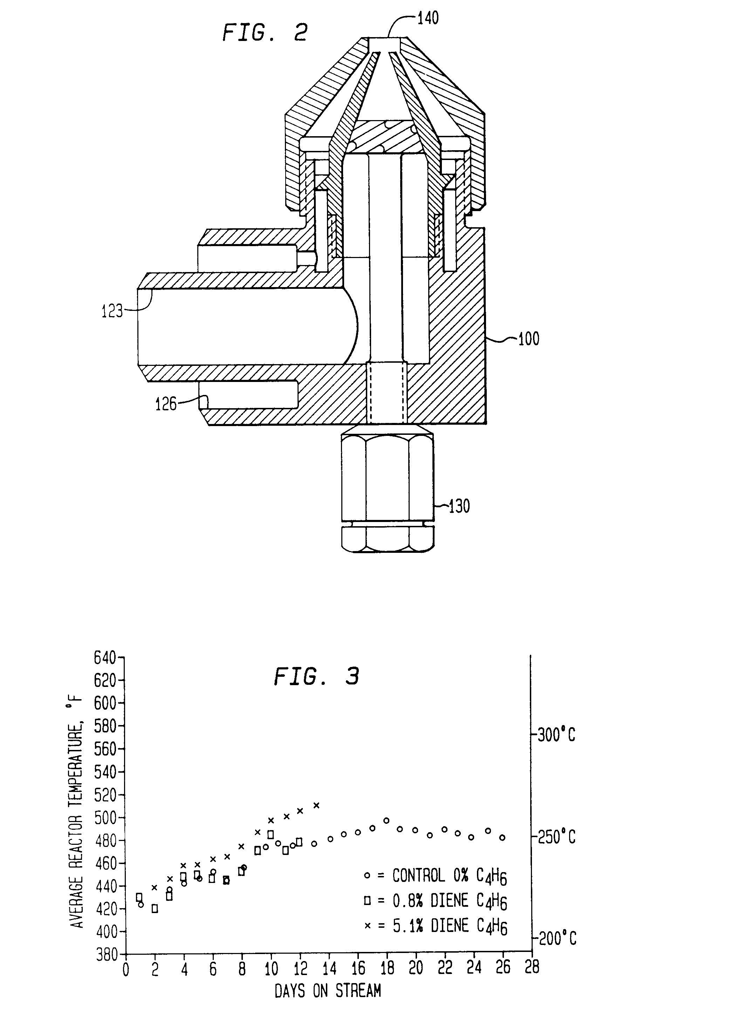

Initial fluidization is achieved by forcing a lift gas upwardly through the catalyst, a light aliphatic $C_4^-$ gas, with or without diluent or recycle, may be charged through inlet port 20A at a bottom portion of the reactor in open communication with chamber 24 beneath grid 8. Pressurized feedstock is introduced above reactant distributor grid 8 via supply conduit 21, pump 22 and distributor conduit 23 to one or more spray nozzle means, described and depicted in Fig. 2. The liquid is dispersed into the bed of catalyst thereabove at a velocity sufficient to form a generally upward flowing suspension of atomized liquid reactant with the catalyst particles and lift gas.

Advantageously, the liquid diene-containing reactant feed is injected into the catalyst bed by atomizing the pressurized liquid feedstream to form readily dispersible liquid particles having an average size of 300 microns or less. This contributes to rapid vaporization of the liquid at process pressure. Exothermic conversion provides sufficient heat to vaporize the liquid quickly, thus avoiding deleterious liquid phase reactions of the diene components, which tend to form carbonaceous deposits such as heavy coke, gums, etc.

A plurality of sequentially connected cyclone separator means 30, 32 and 34 provided with diplegs 36, 38 and 40 respectively are positioned in an upper portion of the reactor vessel comprising dispersed catalyst phase 28.

The product effluent separated from catalyst particles in the cyclone separating system then passes to a plenum chamber 42 before withdrawal via conduit 46, operatively connect with effluent separation system 50. The product effluent is cooled and separated to recover $C_5^-$ liquid hydrocarbons, gaseous recycle or offgas, along with any byproduct water or catalyst fines carried over. A portion of the light gas effluent fraction may be recycled by compressing to form a motive gas for the liquid feed or via recycle conduit 208 for use as lift gas. The recovered hydrocarbon product comprising $C_5^+$ olefins and/or % aromatics, paraffins and naphthenes is thereafter processed as required to provide a desired gasoline or higher boiling product.

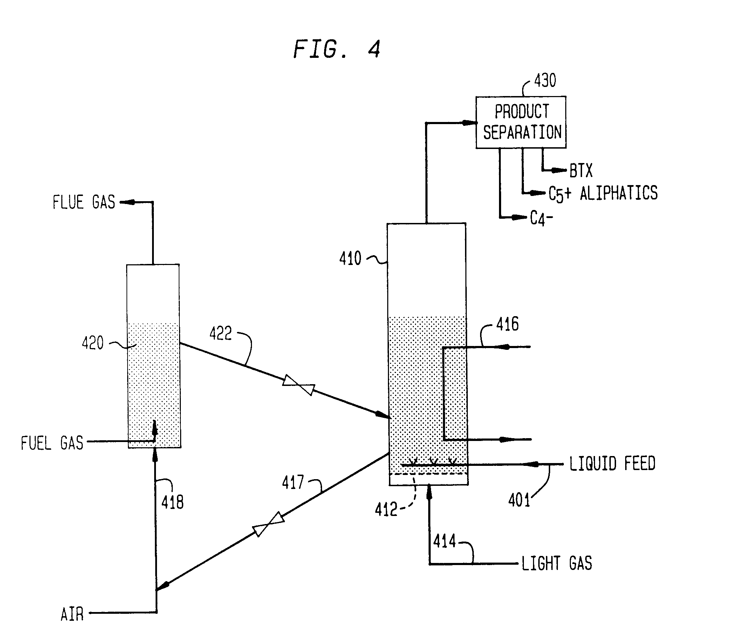

Referring now to Fig. 4, in which maximum BTX production is sought, liquid feedstock is passed at high pressure via feed conduit 401 for injection into vertical reactor vessel 410 above a feed distributor grid 412, which provides for distribution of a lift gas passing via conduit 414 through the small diameter holes in the grid 412. Fluidization is effected in the bottom portion of the bed by upwardly flowing lift gas introduced via conduit 414. Although depicted without baffles, the vertical reaction zone can contain open end tubes above the grill for maintaining hydraulic constraints, as in US Pat. 4,251,484. Optionally, a variety of horizontal baffles may be added to limit axial mixing in the reactor. Thermodynamic conditions in the reaction vessel can be controlled by adjusting feed temperature, catalyst temperature and rate, or by heat exchange means 16. In the reactor configuration shown the heat exchanger tubes limit mixing in the reactor.

Provision is made for withdrawing catalyst from above grid 412 by conduit means 417 provided with flow control valve means to control passage via air lift line 418 to the catalyst regeneration system in vessel 420 where coked catalyst particles are oxidatively regenerated in contact with air or other regeneration gas at high temperature. In order to add sufficient heat to the catalytic reaction zone 410, energy may be added by combustion of flue gas or other fuel stream in the regenerator. Regenerated catalyst is returned to the reactor fluid bed 410 through conduit means 422 provided with flow control valve means. The hot regenerated catalyst is charged to the catalyst bed sufficiently below the upper interface to achieve good mixing in the fluid bed. The rate of flow for regenerated catalyst may be adjusted to provide the degree of thermal input requied for effecting endothermic conversion, and the rate will depend upon the amount and composition of the alkane components.

Initial fluidization is achieved by forcing a lift gas upwardly through the catalyst. A light gas, with or without diluent or recycle, may be charged at a bottom portion of the reactor beneath grid 412. Pressurized liquid feedstock is introduced above reactant distributor grid 412, and pumped to one or more spray nozzle means. The liquid is dispersed into the bed of catalyst thereabove at a velocity sufficient to form a generally upwardly flowing suspension of atomized liquid reactant with the catalyst particles and lift gas.

Cyclone separator means may be positioned in an upper portion of the reactor vessel. The product effluent separated from catalyst particles in the cyclone separating system then passes to effluent separation system 430. The product effluent is cooled and separated to recover C5+ liquid hydrocarbons, gaseous recycle or offgas, along with any byproduct water or catalyst fines carried over. A portion of the light gas effluent fraction may be recycled by compressing to form a motive gas for the liquid feed or recycle for use as lift gas. The recovered hydrocarbon product comprising $C_5^+$ olefins and/or aromatics, paraffins and naphthenes is thereafter processed as described hereafter to provide a desired aromatic product.

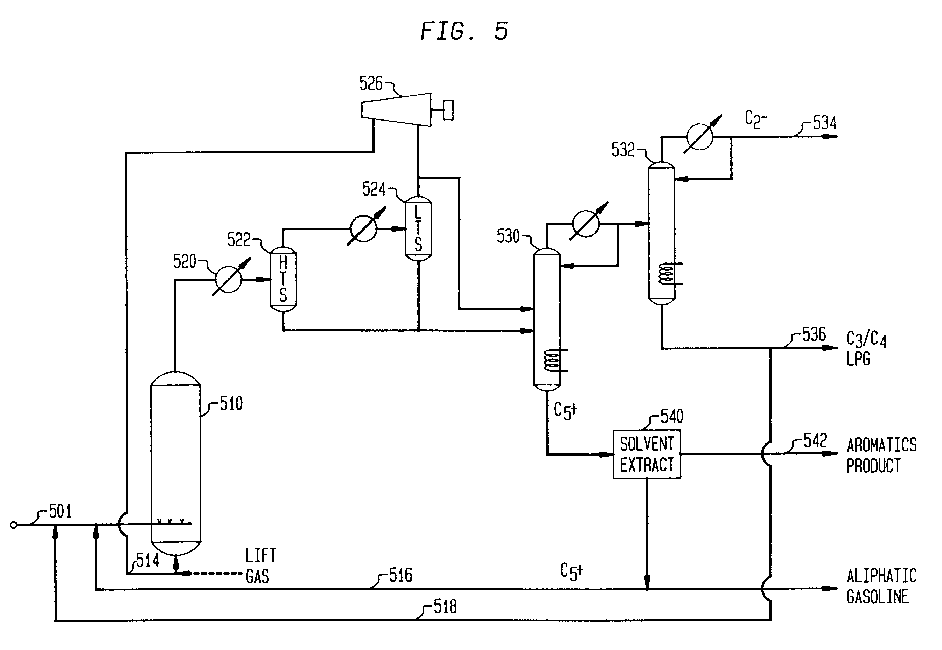

In Fig. 5, directed to maximum recovery of BTX, the feedstock stream 501 is injected reactor vessel 510 containing the fluidized bed of catalyst, along with fluidizing gas stream 514 and recycle streams 516, 518. Reactor effluent is cooled in heat exchanger 520 and partially separated in a series of phase separation drums, HTS 522 (high temperature separator) and LTS 524 (low temperature separator). A light gas stream may be recovered from LTS 524, pressurized in a compressor 528, and recycled via conduit 514 to comprise at least a portion of the lift gas. Condensed liquid from the separators 522, 524 is fed to a debutanizer tower 530, along with a portion of the LTS overhead vapor. Debutanizer overheat vapor is further fractionated by deethanizer tower 532 from which offgas stream 534 is recovered. This light hydrocarbon stream may be employed as fuel gas in the regenerator vessel. Deethanizer liquid bottoms, rich in C3-C4 LPG alkanes, may be recovered via line 536 as product or recycled for further conversion via conduit 518 to reactor 510. The C5+ liquid from the debutanizer 530 is passed to a liquid-liquid extraction unit 540 for recovery of the aromatics components with a selective solvent, such as sulfolane, etc. Following fractionation of the solvent phase, an aromatics product stream 542 is recovered, containing at least 50% BTX. The C5+ aliphatics component may be recovered as a product gasoline stream; however, it is advantageous to recycle this stream for further conversion to increase the net aromatic product.

Under optimized process conditions the turbulent bed has a superficial vapor velocity of about 0.3 to 2 meters per second (m/sec). At higher velocities entrainment of fine particles may become excessive and beyond 10 m/sec the entire bed may be transported out of the reaction zone. At lower velocities, the formation of large bubbles or gas voids can be detrimental to conversion. Even fine particles cannot be maintained effectively in a turbulent bed below about 0.1 m/sec.

A convenient measure of turbulent fluidization is the bed density. A typical turbulent bed has an operating density of about 100 to 500 kg/m$^3$, preferably 300 to 500, measured at the bottom of the reaction zone, becoming less dense toward the top of the reaction zone due to pressure drop and particle size differentiation. This density is generally between the catalyst concentration employed in dense beds and the dispersed transport systems. Pressure differential between two vertically spaced points in the reactor column can be measured to obtain the average bed density at such a portion of the reaction zone. For instance, in a fluidized bed system employing ZSM-5 particles having a clean apparent density of 1.06 gm/cc and packed density of 0.85, an average fluidized bed density of about 300/500 kg/m$^3$ is satisfactory.

By virtue of the turbulence experienced in the turbulent regime, gas-solid contact in the catalytic reactor is improved, providing substantially complete conversion, enhanced selectivity and temperature uniformity. One main advantage of this technique is the inherent control of bubble size and characteristic bubble lifetime. Bubbles of the gaseous reaction mixture are small, random and short-lived, thus resulting in good contact between the gaseous reactants and the solid catalyst particles.

A significant different between the process of this invention and conversion processes of the prior art is that operation in the turbulent fluidization regime is optimized to produce high octane $C_5^+$ liquid in good yield. The weight hourly space velocity and uniform contact provides a close control of contact time between vapor and solid phases, typically about 3 to 25 seconds. Another advantage of operating in such a mode is the control of bubble size and life span, thus avoiding large scale gas by-passing in the reactor. The process of the present invention does not rely on internal baffles in the reactor for the purpose of bubble size control such as the baffles which are employed in the prior art dense bed processes discussed above.

As the superficial gas velocity is increased in the dense bed, eventually slugging conditions occur and with a further increase in the superficial gas velocity the slug flow breaks down into a turbulent regime. The transition velocity at which this turbulent regime occurs appears to decrease with particle size. The turbulent regime extends from the transition velocity to the so-called transport velocity, as described by Avidan et al in U.S. Patent 4,547,616 and by Tabak et al. in U.S. Patent 4,579,999. As the transport velocity is approached, there is a sharp increase in the rate of particle carryover, and in the absence of solid recycle, the bed could empty quickly.

Several useful parameters contribute to fluidization in the turbulent regime in accordance with the process of the present invention. When employing a ZSM-5 type zeolite catalyst in fine powder form such a catalyst should comprise the zeolite suitably bound or impregnated on a suitable support with a solid density (weight of a representative individual particle divided by its apparent "outside" volume) in the range from 0.6-2 g/cc, preferably 0.9-1.6 g/cc. The catalyst particles can be in a wide range of particle sizes up to about 250 microns, with an average particle size between about 20 and 100 microns, preferably in the range of 10-150 microns and with the average particle size between 40 and 80 microns. When these solid particles are placed in a fluidized bed where the superficial fluid velocity is 0.3-2 m/s, operation in the turbulent regime is obtained. The velocity specified here is for an operation at a total reactor pressure of about 100 to 300 kPa. Those skilled in the art will appreciate that at higher pressures, a lower gas velocity may be employed to ensure operation in the turbulent fluidization regime.

The reactor can assume any technically feasible configuration, but several important criteria should be considered. The bed of catalyst in the reactor can be at least about 5-20 meters in height, preferably about 7 meters. Fine particles may be included in the bed, especially due to attrition, and the fines may be entrained in the product gas stream. A typical turbulent bed may have a catalyst carryover rate up to about 1.5 times the reaction zone inventory per hour. If the fraction of fines becomes large, a portion of the carryover can be removed from the system and replaced by larger particles. It is feasible to have a fine particle separator, such as a cyclone disposed within the reactor shell to recover catalyst carryover and return this fraction continuously to the bottom of the reaction zone for the recirculation at a rate of about one catalyst inventory per hour. Optionally, fine particles carried from the reactor vessel entrained with effluent gas can be recovered by a high operating temperature sintered metal filter.

This process can be used with any process stream which contains sufficient liquid olefins and dienes. Preferably the feed is substantially free of deleterious oxygenates and sulfur compounds. Because the catalyst can be readily removed and regenerated, or replaced, the process can tolerate a lot of impurities in the feed. Experimental runs are performed using a ZSM-5 catalyst to demonstrate the inventive process. The fluidized bed unit can be operated over a wide range of process variables and catalyst activity.

Reactor Operation

A typical single pass reactor unit employs a temperature-controlled catalyst zone with indirect heat exchange and/or adjustable gas quench, whereby the reaction exotherm can be carefully controlled to prevent excessive temperature above the usual operating range of about 315°C to 650°C. Preferably average reactor temperature is 340°C to 430°C to maximize production of gasoline boiling range hydrocarbons. Temperatures of 425-580°C maximize production of aromatics. Heat exchanging hot reactor effluent with feedstock and/or recycle streams will save energy. Optional heat exchangers may recover heat from the effluent stream prior to fractionation. It is preferred to operate the olefin conversion reactors at moderate pressure of about 100 to 3000 kPa (atmospheric to about 400 psig) to maximize liquid yields. Higher pressures, to 6000 kPa, favor aromatics yields.

The weight hourly space velocity (WHSV, based on total olefins in the fresh feedstock is about 0.1-5 WHSV. Typical product fractionation systems are described in U.S. Patents 4,456,779 and 4,504,693 (Owen, et al.).

To prevent premature non-catalytic reaction of the dienes, it is desirable to maintain reactant liquid feedstream temperature below about 180°C (350°F) until injection into the fluidized bed. Appropriate thermal insulation or quenching of the feedstream to the injection point can largely prevent gum and coke formation in the liquid phase prior to catalysis.

Atomization of the pressurized liquid reactant feedstream can be achieved by known techniques, such as liquid spray nozzles, motive gas, ultra sonics, etc. A suitable nozzle is shown in Fig. 2, wherein a concentric feed liquid projection device 100 is depicted in vertical cross section view. Pressurized liquid flows through a supply conduit 123. The nozzle is mounted onto the vessel internal structure by screw cap means 130 or similar attachment means. A motive fluid supplied under pressure through conduit 126 drives the pressurized liquid flowing from the nozzle orifice 140 for injection into the reaction vessel at sufficient velocity to induce a fine vertically directed spray of atomized liquid having an average particle size up to about 300, preferably about 50 microns. The number and arrangement of nozzles will depend upon the cross sectional area of the fluidized bed and fluidization characteristics of the gas-solid-liquid mixture. The atomized stream from a pressurized nozzle can be made to effect penetration into the bed at a depth and/or lateral radius of a meter or more. The mixture fluid may be an inert material, nitrogen, lower aliphatic gas, stream, etc.

Thermal insulation of the liquid diene-containing feedstream from the hot reaction medium in the reaction vessel can be achieved by applying to the liquid feed conduit a layer of thermal insulation, such as a ceramic shield or the like. Jacketed conduits with heat absorbing fluid may also be suitable.

EXAMPLE 1

In the present example $C_4^+$ liquid stream is converted to aromatics-rich gasoline in the fluidized bed reactor employing acid ZSM-5 powder catalyst having a fresh alpha value of about 80 at an average conversion temperature of about 425°C (800°F) and total pressure of about 275 kPa (25 psig).

The liquid pyrolysis gasoline feedstock contains about 22 wt. % $C_4^+$ mono-alkenes, 27% $C_4^+$ dienes (mainly 1,3-butadiene), 49% $C_4^+$ paraffins, 2% aromatics and naphthenes, and less than 1% $C_3^-$ aliphatics.

Typical olefinic pyrolysis byproduct streams are shown in Table 1.

Following initial heating and fluidization of the powdered catalyst with a heated lift gas (e.g. $C_2^-$ hydrocarbon), the feedstream is preheated and maintained below 180°C prior to injection into the bed. After achieving steady state operation at a reaction severity index (R.I.) of about 1, the effluent conversion product (less any lift gas components) comprises 82 wt. % $C_5^+$ liquid gasoline having a research octane rating of 94 (RON). The total aromatics content is 18 wt. %, including 1% benzene (B), 5% toluene (T), 6% xylenes (X) and ethyl benzene, 4% $C_9$ aromatics isomers and 10% $C_{10}$ isomers, mainly durene. The predominant nonaromatic fraction (65%) contains mainly mono-olefins, parafins and naphthenes, and the light gas $C_4^-$ fraction is 17% of the conversion product.

TABLE 1

| Example of Diene-Rich Feedstock (ethane cracker byproduct) | |
|---|---|
| Component | Vol. % |
| $C_3-$ | 1.0 |
| i-butene | 0.08 |
| 1,3-butadiene | 0.51 |
| t.2,butene | 0.1 |
| c.2,butene | 0.15 |
| 1,2 butadiene | 0.14 |
| 3m 1 butene | 0.45 |
| isopentane | 5.44 |
| 1,4 pentadiene | 0.6 |
| 1-pentene | 0.63 |
| n-pentane | 1.92 |
| isoprene | 2.3 |
| c,2,pentene | 0.35 |
| 2m2butene | 0.45 |
| t,1,3, pentadiene | 1.5 |
| c,1,3,pentadiene | 1.0 |
| cyclopentadiene | 13.7 |
| cyclopentene | 1.7 |
| 2,3 d.m. butane | 1.7 |
| 3mpentene | 0.85 |
| hexane | 0.95 |
| unknown $C_6$ | 1.04 |
| cyclohexane | 3.06 |
| benzene | 34.4 |
| unknown $C_8$ | 3.47 |
| Toluene | 10.1 |
| vinyleydohexene | 0.19 |
| ethylbenzene | 1.29 |
| xylene | 1.01 |
| styrene | 0.3 |
| unknown $C_9$ + | 6.9 |

The above diene-rich stream example contains $C_6^+$ aromatic hydrocarbons which can be separated before feeding to the reactor. Typical ranges of diene-rich pyrolysis gasoline streams comprised of mainly $C_4$-$C_6$ hydrocarbons are:

| | Vol. % |
|---|---|
| Dienes | 5-60 |
| Mono-alkenes | 5-30 |
| Aromatics | 1-5* |
| Alkanes | 20-60 |
| Naphthenes | 1-5 |

*can be as high as 60% if $C_6^+$ fraction is not separated.

EXAMPLES 2-4

A series of continuous olefin conversion runs are conducted using H-ZSM-5 (65%) catalyst having an alpha value of about 175 at the beginning of the aging runs made under oligomerization conditions without regeneration to upgrade mixtures of ethene, propene and butadiene and to determine the effects of diene concentration on catalyst aging. The control feedstock (Example 2) is compared with diene-containing feeds in Table 1.

TABLE 2

|  | Example 2 | Example 3 | Example 4 |
|---|---|---|---|
| Ethene | 0 | 0.7 | 1.8 |
| Propene | 26.8 | 28.1 | 22.9 |
| Butenes | 35.7 | 31.9 | 31.7 |
| 1,3 Butadiene | 0 (control) | 0.8 | 5.1 |
| Alkanes ($C_4{}^-$) | 37.5 | 38.5 | 38.5 |
| Recycle (mol/mol olefin) | 2.5:1 | 2.5:1 | 2.5:1 |

The conversion unit is a single bed isothermal reactor using HZSM-5 having a crystal size less than 0.5 microns, together with 35% alumina binder and having a fresh alpha value of 175. The continuous runs are conducted at about 6600 kPa and weight hourly space velocity (WHSV) of about 0.8 parts olefin feed per part by weight of catalyst per hour. The conversion runs are started at 205°C (400°F) and the temperature is increased to compensate for coke deposition, while maintaining total olefin conversion of at least 80%, preferably over 90%. Results of the aging studies are plotted in Fig. 3, with all conversion rates being normalized to 80% to 166°C + (330°F +) product for comparison purposes. Selectivity of the conversion product to heavier hydrocarbons is shown in Table 3.

TABLE 3

|  | Example 2 | Example 3 | Example 4 |
|---|---|---|---|
| Total Liquid Product, 50% pt, °C (°F) | 261/(501) | 259/(498) | 244/(472) |
| Distillate Species (As Cut) | | | |
| 5 wt. %, °C (°F) | 232/(434) | 250/(483) | 297(477) |
| 95 wt. % °C (°F) | 369/(697) | 383/(722) | 379(715) |
| Gravity, °API | 44.3 | 41.2 | 38.9 |
| Aniline Point | 177 | 184 | 172 |

While the aromatics product content of the control runs averaged about 2-5%, the 5.1% butadiene feed (Example 4) is upgraded to an aromatics content of 15.5 wt. %, more than 3 times the diene input. The average paraffin content is less than 14% and the liquid dominant product is 70% + olefins and naphthenes.

These results indicate butadiene, at levels of 1 wt. % or less, do not cause significantly increased catalyst aging or lower product selectivity. It was surprising that adding diene would increase the aromatics yield, and surprising that such large aromatics yields could be achieved at reactor temperatures just above 200°C. Typical FCC $C_3/C_4$ olefins from a depropanizer feed stream contain 0.3-0.6 wt. % butadiene which is less than the 0.8 wt. % butadiene concentration that was used in this study. Even at the 5.1 wt. % butadiene level, though catalyst aging was increased, product selectivity to heavier hydrocarbons remained relatively high.

EXAMPLE 5

10

In the present high severity example a $C_4^+$ liquid stream is converted to aromatics-rich gasoline in the fluidized bed reactor employing acid ZSM-5 powder catalyst having a fresh alpha value of about 175 at an average conversion temperature about 480°C (900°F) and total pressure of about 275 kPa (25 psig).

The liquid feed was the same as used in example 1.

Following initial heating and fluidization of the powdered catalyst with a heated lift gas (e.g. $C_2^-$ hydrocarbon), the feedstream is preheated and maintained below 180°C prior to injection into the bed. After achieving steady state operation at a reaction severity index (R.I.) of about 2, the effluent conversion product (less any lift gas components) has an aromatics content of 34.4 wt. %, including 4.9% Benzene (B), 11.8% toluene (T), 14% xylenes (X) and 0.9% ethyl benzene, 2.3% $C_9$ aromatics isomers and 0.5% $C_{10}$ isomers. The nonaromatic fraction contains mainly mono-olefins, paraffins and naphthenes, and the light gas $C_4^-$ fraction is 13.5% of the conversion product.

Comparative effluent streams for high severity and low severity conversion runs under steady state reactor conditions are shown in Table 4. High severity operation means a reactor temperature of 600°C. Low severity operation was run at 425°C reactor temperature, at a RI of 2. Both were run at 0.87 WHSV. 1 bar (100 kPa) over HZSM-5.

TABLE 4

| Products Yield | EXAMPLE 5A High Severity | EXAMPLE 5B Low Severity |
|---|---|---|
| $H_2$, wt. % | 3.0 | 1.3 |
| $C_1$ | 13.5 | 2.6 |
| $C_2$ | 10.3 | 6.1 |
| $C_3$ | 3.8 | 3.4 |
| $C_4$ | 2.0 | 1.4 |
| $C_5$ Non-Aromatic | - | 50.3 |
| Benzene | 22.1 | 4.9 |
| Toluene | 21.9 | 11.8 |
| Ethyl Benzene | 0.9 | 0.9 |
| Xylene | 15.6 | 14.0 |
| $C_9$ Aromatics | 2.7 | 2.3 |
| $C_{10}^+$ | 3.2 | 0.5 |
| Coke | 1.0 | 0.5 |
| | $\overline{100.0}$ | $\overline{100.0}$ |

The flexibility of the fluid bed operating parameters for controlling the reactor temperature under exothermic reaction conditions allows an easy adjustment for achieving the optimal yield structure.

Either $C_5$ + liquid yield, or BTX yield may be optimized when temperatures of 315-650°C are used in the reactor. The net yield of aromatics can comprise over 30% of the olefins in the feed. The propane:propene ratio will usually be 0.7:1 to 5:1 to maximize aromatics.

To fluidize the catalyst at the bottom of the reactor prior to injection of the liquid feed stream, a lift gas may be employed. This can be an inert diluent or recycled light gas, such as methane, ethane, ethene, propane, etc. Recycle of $C_3^-$ light hydrocarbons may also be desirable under certain circumstances, for instance with unreacted aliphatics which require further conversion or for dilution of highly exothermic feedstocks.

The thermodynamic balance of exothermic olefin oligomerization and endothermic paraffin reactions can have significant impact on the reaction severity conditions.

The use of fluid-bed reactor in this process offers several advantages over a fixed-bed reactor. Due to continuous catalyst regeneration, fluid-bed reactor operation need not be adversely affected by oxygenate, sulfur and/or nitrogen containing contaminants present in the pyrrolysis byproduct.

**Claims**

1. A process for converting an olefinic feed containing 5 to 90 wt% $C_4$-$C_6$ olefins and at least 1 wt% dienes to a liquid product, of reduced diene content, comprising $C_4$ + hydrocarbons and having a $C_3$-$C_5$ alkane:alkene weight ratio of 0.2 to 200, said process comprising contacting said feed with a gas-fluidized bed of a catalyst comprising a zeolite having a constraint index of 1 to 12 at a temperature of 220 to 510°C and a reaction severity index, measured as the weight ratio of propane to propene in reactor effluent, below 50, the feed being at a temperature below that of the bed before introduction thereinto.

**2.** A process according to claim 1 wherein the fluidized bed is maintained as a turbulent fluidized bed having a density of 100 to 500 kg/m$^3$ at its bottom.

**3.** A process according to claim 1 or claim 2 wherein the zeolite is ZSM-5.

**4.** A process according to any preceding claim wherein the zeolite has an activity, alpha, of 10 to 250.

**5.** A process according to any preceding claim wherein 10 to 25 wt% of said catalyst is in the form of particles of size less than 32 micrometers.

**6.** A process according to any preceding claim wherein said feed is introduced into said bed as an atomized stream of liquid.

**7.** The process of any preceding claim further characterized in that the superficial feed vapor velocity is 0.3-2 m/sec; the bed temperature is 315 to 510°C; the weight hourly feedstock space velocity (based on total olefin ) is 0.1 to 5; the propane:propene weight ratio of reactor effluent is 0.7:1 to 2:1; and the average fluidized bed density measured at the reaction zone bottom is 300 to 500 kg/m$^3$.

**8.** The process of any preceding claim further characterized in that a $C_3$- hydrocarbon gas is added to the feed.

**9.** The process of any preceding claim further characterized in that a portion of light hydrocarbon gas is recovered from reactor effluent and recycled to a lower portion of the reactor as a fluidizing lift gas added to the reactor below the liquid feed.

**10.** The process of any preceding claim further characterized in that the catalyst contains a hydogenation-dehydrogenation metal component to increase aromatics production.

**11.** The process of any preceding claim further characterized in that the hydrocarbon product is fractionated to produce an aromatics-rich $C_5$ + liquid stream, and the aromatics are extracted from the $C_5$ + liquid to produce a product stream comprising at least 50 wt % $C_6$-$C_{10}$ aromatics, and a raffinate rich in non-aromatics.

**12.** The process of claim 11 further characterized in that at least a portion of the raffinate is recycled to the fluidized bed.

**13.** The process of any preceding claim further characterized in that a portion of the fluidized catalyst is at least intermittently withdrawn, regenerated, and returned to the fluidized bed.

**14.** The process of claim 13 further characterized in that a portion of the catalyst is continuously regenerated with a gas comprising oxygen to produce hot regenerated catalyst which is recycled to the fluidized bed and heats it.

**Revendications**

**1.** Un procédé pour la conversion d'une charge oléfinique, contenant 5 à 90% en poids d'oléfines en $C_4$-$C_6$ et au moins 1% en poids de diènes, en un produit liquide, à teneur réduite en diènes, comprenant des hydrocarbures en $C_4$ + et présentant une proportion pondérale alcane en $C_3$-$C_5$/alcène de 0,2 à 200, ce procédé consistant à mettre en contact ladite charge avec un lit fluidisé par un gaz, d'un catalyseur constitué d'une zéolite ayant un indice de contrainte de 1 à 12 à une température de 220 à 510°c, et un indice de sévérité de la réaction, mesuré par la proportion pondérale du propane au propène dans l'effluent du réacteur, inférieur à 50, la charge se trouvant à une température inférieure à celle du lit avant son introduction dans ce dernier.

**2.** Un procédé selon la revendication 1, dans lequel le lit fluidisé est maintenu sous forme d'un lit fluidisé turbulent ayant à sa partie inférieure une masse volumique de 100 à 500 kg/m$^3$.

3. Un procédé selon la revendication 1 ou la revendication 2, dans lequel la zéolite est la zéolite ZSM-5.

4. Un procédé selon l'une quelconque des revendications précédentes, dans lequel la zéolite a un indice d'activité alpha de 10 à 250.

5. Un procédé selon l'une quelconque des revendications précédentes, dans lequel 10 à 25% en poids dudit catalyseur se présentent sous forme de particules ayant une granulométrie inférieure à 32 $\mu$m.

6. Un procédé selon l'une quelconque des revendications précédentes, dans lequel ladite charge est introduite dans ledit bain sous forme d'un courant atomisé de liquide.

7. Le procédé selon l'une quelconque des revendications précédentes, caractérisé en outre en ce que la vitesse superficielle de la vapeur de charge est de 0,3 à 2 m/s; la température du lit est de 315 à 510˚ C; la vitesse spatiale horaire de la charge (par rapport au total des oléfines) est de 0,1 à 5 h$^{-1}$; la proportion pondérale propane/propène dans l'effluent du réacteur est de 0,7/1 à 2/1; et la masse volumique moyenne du lit fluidisé, mesurée dans la partie inférieure de la zone de réaction, est de 300 à 500 kg/m$^3$.

8. Le procédé selon l'une quelconque des revendications précédentes, caractérisé en outre en ce qu'on ajoute à la charge un hydrocarbure gazeux en C$_3$-.

9. Le procédé selon l'une quelconque des revendications précédentes, caractérisé en outre en ce qu'une partie des hydrocarbures gazeux légers est récupérée de l'effluent du réacteur et recyclée vers une partie inférieure du réacteur, tandis qu'un gaz ascensionnel de fluidisation est introduit dans le réacteur en-dessous du liquide de charge.

10. Le procédé selon l'une quelconque des revendications précédentes, caractérisé, en outre, en ce que catalyseur contient un composant métallique d'hydrogénation-déshydrogénation pour augmenter la production des aromatiques.

11. Le procédé selon l'une quelconque des revendications précédentes, caractérisé en outre en ce que l'hydrocarbure produit est fractionné pour donner un courant liquide en C$_5$ + riche en aromatiques, et les aromatiques sont extraits du liquide en C$_5$ + pour donner un courant de produit comprenant au moins 50% en poids d'aromatiques en C$_6$-C$_{10}$ et un raffinat riche en non-aromatiques.

12. Le procédé selon la revendication 11, caractérisé en outre en ce qu'au moins une partie du raffinat est recyclée vers le lit fluidisé.

13. Le procédé selon l'une quelconque des revendications précédentes, caractérisé en outre en ce qu'une partie du catalyseur fluidisé est, au moins d'une manière intermittente, soutirée, régénérée et renvoyée au lit fluidisé.

14. Le procédé selon la revendication 13, caractérisé en outre en ce qu'une partie du catalyseur est régénérée en continu avec un gaz comprenant de l'oxygène, pour produire un catalyseur régénéré chaud, qui est recyclé vers le lit fluidisé et le chauffe.

**Patentansprüche**

1. Verfahren zur Umwandlung einer Olefinzufuhr, die 5 bis 90 Gew% C$_4$-C$_6$-Olefine und mindestens 1 Gew% Diene enthält, zu einem flüssigen Produkt mit reduziertem Diengehalt, das C$_4^+$-Kohlenwasserstoffe enthält und ein Gewichtsverhältnis von C$_3$-C$_5$-Alkan:Alken von 0,2 bis 200 aufweist, wobei das Verfahren den Kontakt dieser Zufuhr mit einem mit Gas aufgewirbelten Bett eines Katalysators, der einen Zeolith mit einem Zwangsindex von 1 bis 12 umfaßt, bei einer Temperatur von 220 bis 510˚ C und einem Severity-Index, gemessen als Gewichtsverhältnis des Propan zum Propen im Reaktorabfluß, von weniger als 50 umfaßt, wobei die Zufuhr vor der Einführung in das Bett bei einer Temperatur unterhalb der des Bettes ist.

13

**2.** Verfahren nach Anspruch 1, worin das Wirbelschichtbett als Turbulenz-Wirbelschichtbett mit einer Dichte von 100 bis 500 kg/m$^3$ an der Unterseite gehalten wird.

**3.** Verfahren nach Anspruch 1 oder 2, worin der Zeolith ZSM-5 ist.

**4.** Verfahren nach einem der vorstehenden Ansprüche, worin der Zeolith eine Aktivität, Alpha, von 10 bis 250 aufweist.

**5.** Verfahren nach einem der vorstehenden Ansprüche, worin 10 bis 25 Gew% des Katalysators in Form von Partikeln mit einer Größe von weniger als 32 $\mu$m vorliegen.

**6.** Verfahren nach einem der vorstehenden Ansprüche, worin die Zufuhr als atomisierter Strom der Flüssigkeit in das Bett eingeführt wird.

**7.** Verfahren nach einem der vorstehenden Ansprüche, weiterhin dadurch gekennzeichnet, daß die Oberflächengeschwindigkeit der Dampfzufuhr 0,3 bis 2 m/s, die Bettemperatur 315 bis 510°C, die stündliche Gewichts-Zufuhr-Raum-Geschwindigkeit (auf das gesamte Olefin bezogen) 0,1 bis 5, das Gewichtsverhältnis Propan:Propen des Reaktorabflusses 0,7:1 bis 2:1 und die durchschnittliche Wirbelschichtdichte, gemessen am Boden der Reaktionszone, 300 bis 500 kg/m$^3$ betragen.

**8.** Verfahren nach einem der vorstehenden Ansprüche, weiterhin dadurch gekennzeichnet, daß der Zufuhr ein $C_3$-Kohlenwasserstoffgas zugesetzt wird.

**9.** Verfahren nach einem der vorstehenden Ansprüche, weiterhin dadurch gekennzeichnet, daß ein Teil des Kohlenwasserstoffleichtgases aus dem Reaktorabfluß gewonnen und zum unteren Abschnitt des Reaktors als fluidisierendes Auftriebsgas rezirkuliert wird, das dem Reaktor unterhalb der Flüssigkeitszufuhr zugesetzt wird.

**10.** Verfahren nach einem der vorstehenden Ansprüche, weiterhin dadurch gekennzeichnet, daß der Katalysator eine Hydrierungs/Dehydrierungs-Metallkomponente enthält, um die Erzeugung der Aromaten zu steigern.

**11.** Verfahren nach einem der vorstehenden Ansprüche, weiterhin dadurch gekennzeichnet, daß das Kohlenwasserstoffprodukt fraktioniert wird, um einen flüssigen $C_5^+$-Strom zu erzeugen, der reich an Aromaten ist, und die Aromaten von der $C_5^+$-Flüssigkeit extrahiert werden, um einen Produktstrom, der mindestens 50 Gew-% $C_6$-$C_{10}$-Aromaten enthält, und ein Raffinat zu erzeugen, das reich an Nichtaromaten ist.

**12.** Verfahren nach Anspruch 11, weiterhin dadurch gekennzeichnet, daß zumindest ein Teil des Raffinates zum Wirbelschichtbett rezirkuliert wird.

**13.** Verfahren nach einem der vorstehenden Ansprüche, weiterhin dadurch gekennzeichnet, daß ein Teil des fluidisierten Katalysators zumindest diskontinuierlich abgezogen, regeneriert und zum Wirbelschichtbett zurückgeführt wird.

**14.** Verfahren nach Anspruch 13, weiterhin dadurch gekennzeichnet, daß ein Teil des Katalysators kontinuierlich mit einem Gas regeneriert wird, das Sauerstoff umfaßt, um einen heißen regenerierten Katalysator zu erzeugen, der zum Wirbelschichtbett rezirkuliert wird und dieses erwärmt.

14

# FIG. 1

FIG. 2

FIG. 3

AVERAGE REACTOR TEMPERATURE, °F

DAYS ON STREAM

o = CONTROL 0% $C_4H_6$
□ = 0.8% DIENE $C_4H_6$
× = 5.1% DIENE $C_4H_6$

# FIG. 4

FIG. 5